# FASCICULE DE BREVET EUROPEEN

(11) **EP 1 611 872 B1**
(45) Date de publication et mention de la délivrance du brevet: **11.11.2009**
(21) Numéro de dépôt: 05356113.0
(22) Date de dépôt: 27.06.2005
(51) Int. Cl.: A61F 2/40, A61F 2/32

(54) **Prothèse d'épaule ou de hanche**
Schulter- oder Hüftprothese
Shoulder or hip prosthesis

(30) Priorité: 28.06.2004 FR 0407071
(43) Date de publication de la demande: 04.01.2006
(73) Titulaire: TORNIER, 38330 Saint-Ismier (FR)
(72) Inventeur: Tornier, Alain, 38330 Saint Ismier (FR); Manceau, Thierry, 38400 Saint Martin d'Heres (FR)
(74) Mandataire: Myon, Gérard Jean-Pierre

(56) Documents cités:
- EP-A- 0 807 426
- EP-A- 0 809 986
- EP-A- 0 864 306
- EP-A- 1 380 274
- FR-A- 2 248 820
- US-A- 3 916 451
- US-A- 4 054 955
- US-A- 4 846 840
- US-B1- 6 312 467

## Description

L'invention a trait à une prothèse totale ou partielle d'épaule ou de hanche permettant de reproduire, avec une bonne précision, les caractéristiques d'une articulation naturelle.

Il est connu de FR-A-2 841 768 de réaliser une prothèse d'épaule ou de hanche comprenant un élément intermédiaire formé d'une coupelle et d'une rondelle définissant deux surfaces convexes destinées à coopérer respectivement avec une surface d'articulation concave formée par un patin relié au composant huméral ou fémoral et une surface d'articulation concave glénoïdienne ou cotyloïdienne, naturelle ou prothétique. Le positionnement du patin entre le composant intermédiaire et le composant huméral ou fémoral a pour effet d'induire un encombrement relativement important de la prothèse selon une direction parallèle à l'axe médian d'une tige reliant ce patin à une partie du composant huméral ou fémoral ancrée dans l'os correspondant. Ceci a pour conséquence un décalage entre l'humérus et la glène ou entre le fémur et le bassin, un tel décalage devant s'avérer, pour ces pathologies, gênant pour le patient.

C'est à ces inconvénients qu'entend plus particulièrement remédier l'invention en proposant une nouvelle prothèse d'épaule ou de hanche grâce à laquelle on peut réduire la distance entre le plan de coupe humérale ou fémorale, d'une part, et la surface articulaire glénoïdienne ou cotyloïdienne, d'autre part.

Dans cet esprit, l'invention concerne une prothèse d'épaule ou de hanche comprenant un premier composant, huméral ou fémoral, présentant une surface d'articulation concave et un deuxième composant, intermédiaire, présentant une première et une seconde surfaces d'articulation convexes destinées à coopérer respectivement avec la surface d'articulation concave du premier composant et avec une surface d'articulation concave glénoïdienne ou cotyloïdienne naturelle ou définie par un troisième composant, glénoïdien ou cotyloïdien, ce deuxième composant étant creux et définissant un volume de réception d'une partie d'un organe appartenant au premier composant, cette partie étant reliée à une partie métaphysaire du premier composant par une tige traversant une ouverture ménagée dans une bague appartenant à ce second composant et montée sur une cupule dont la surface externe forme la seconde surface d'articulation convexe précitée. Cette prothèse est caractérisée en ce que la première surface d'articulation convexe du deuxième composant est formée sur la surface externe de la bague, autour de l'ouverture précitée.

Grâce à l'invention, la première surface d'articulation convexe du composant intermédiaire est formée sur l'extérieur de celui-ci, ce qui lui permet d'interagir avec une surface du premier composant immédiatement voisine de sa partie métaphysaire, voire porté par celle-ci. Il en résulte une bonne compacité de la prothèse qui est bien adaptée à certaines pathologies.

Selon des aspects avantageux mais non obligatoires, une prothèse d'épaule ou de hanche peut incorporer une ou plusieurs des caractéristiques suivantes prises dans toute combinaison techniquement admissible :
- L'ouverture de la bague du second composant est allongée, avec une largeur inférieure à la largeur maximale de la partie de l'organe reçue dans le volume intérieur du second composant, la largeur de cette ouverture étant légèrement supérieure à la largeur de la tige précitée. Grâce à cet aspect de l'invention, la partie de l'organe du premier composant est retenue à l'intérieur du volume du second composant, ce qui assure une bonne tenue de l'assemblage, alors que la largeur de l'ouverture permet un guidage en translation de l'organe du premier composant au niveau de sa tige.
- Selon un premier mode de réalisation, la surface d'articulation concave du premier composant est formée sur une portion de l'organe précité qui est monobloc avec la partie introduite dans le volume intérieur du second composant et la tige associée, cet organe étant rapporté sur une partie métaphysaire du premier composant. Selon un autre mode de réalisation, la surface d'articulation concave du premier composant est formée sur une partie métaphysaire du premier composant.
- Quel que soit le mode de réalisation considéré, l'une des surfaces, parmi la première surface convexe du second composant et la surface concave du premier composant, peut être en saillie par rapport au composant auquel elle appartient, alors que l'autre de ces surfaces est en retrait par rapport à l'autre composant, la surface en saillie étant bordée par deux bords globalement parallèles, alors que la surface en retrait est bordée par deux bords globalement parallèles entre lesquels sont disposés les bords de la surface en saillie en configuration montée de la prothèse.
- L'un des premier et deuxième composants forme une glissière en creux de réception et de guidage d'une partie en saillie de l'autre composant, parmi les premier et deuxième composants, la première surface d'articulation convexe du second composant et la surface d'articulation concave du premier composant étant respectivement formées par le fond de cette glissière et par la surface d'extrémité de la partie en saillie, ou inversement.
- L'ouverture de la bague est pourvue d'une zone élargie d'introduction de la partie précitée de l'organe dans le volume de réception du second composant. Cette bague peut être élastiquement déformable au moins au niveau de la zone élargie, de façon à permettre l'introduction de la partie précitée dans le volume précité. Selon une autre approche, la zone élargie peut être ménagée à une extrémité de l'ouverture de la bague qui est allongée, le premier ou le deuxième composant, voire les deux, étant pourvu(s) d'au moins un relief de blocage apte à empêcher l'alignement de cette partie et de cette zone élargie en configuration montée de la prothèse.
- La première surface d'articulation est globalement en forme de tronc de cylindre centré sur un axe antéro-postérieur de l'articulation en configuration montée et implantée de la prothèse.

L'invention sera mieux comprise et d'autres avantages de celle-ci apparaîtront plus clairement à la lumière de la description qui va suivre de trois modes de réalisation d'une prothèse conforme à son principe, donnée uniquement à titre d'exemple et faite en référence aux dessins annexés dans lesquels :
- la figure 1 est une coupe sagittale de principe d'une prothèse d'épaule conforme à l'invention en place sur un patient, alors que le bras du patient est en position intermédiaire ;
- la figure 2 est une coupe analogue à la figure 1 à plus petite échelle, alors que le bras du patient est en position relevée par rapport à celle de la figure 1 ;
- la figure 3 est une coupe analogue à la figure 2, alors que le bras du patient est en position basse ;
- la figure 4 est une vue en perspective éclatée des composants huméral et intermédiaire de la prothèse des figures 1 à 3,
- la figure 5 est une coupe analogue à la figure 1 pour une prothèse de hanche conforme à un second mode de réalisation l'invention ;
- la figure 6 est une coupe selon la ligne VI-VI à la figure 5 ;
- la figure 7 est une vue en perspective éclatée des composants fémoral et intermédiaire de la prothèse des figures 5 et 6 ;
- la figure 8 est une coupe analogue à la figure 1 pour une prothèse d'épaule conforme à un troisième mode de réalisation conforme à l'invention ;
- la figure 9 est une coupe selon la ligne IX-IX à la figure 8 ;
- la figure 10 est une vue en perspective d'une partie du composant intermédiaire et du composant huméral dans la configuration des figures 8 et 9 ;
- la figure 11 est une vue de côté de la prothèse des figures 8 à 10 dans une autre configuration ;
- la figure 12 est une vue en perspective analogue à la figure 10, lorsque la prothèse est dans la configuration de la figure 11 et
- la figure 13 est une vue en perspective éclatée des composants huméral et intermédiaire de la prothèse des figures 8 à 12.

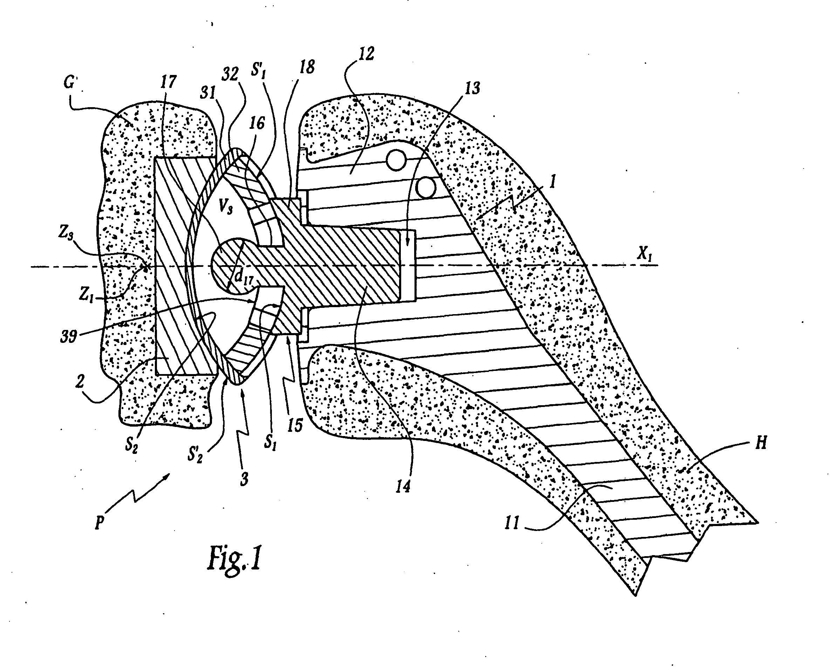

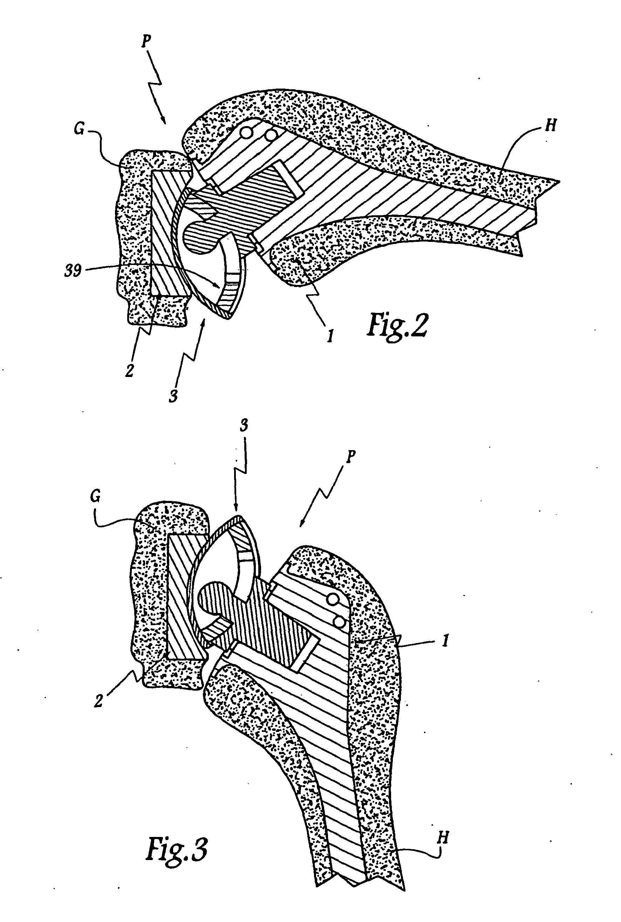

Pour la clarté du dessin, les os dans lesquels sont implantés les composants prothétiques sont représentés uniquement aux figures 1 à 3, 5, 6 et 8.

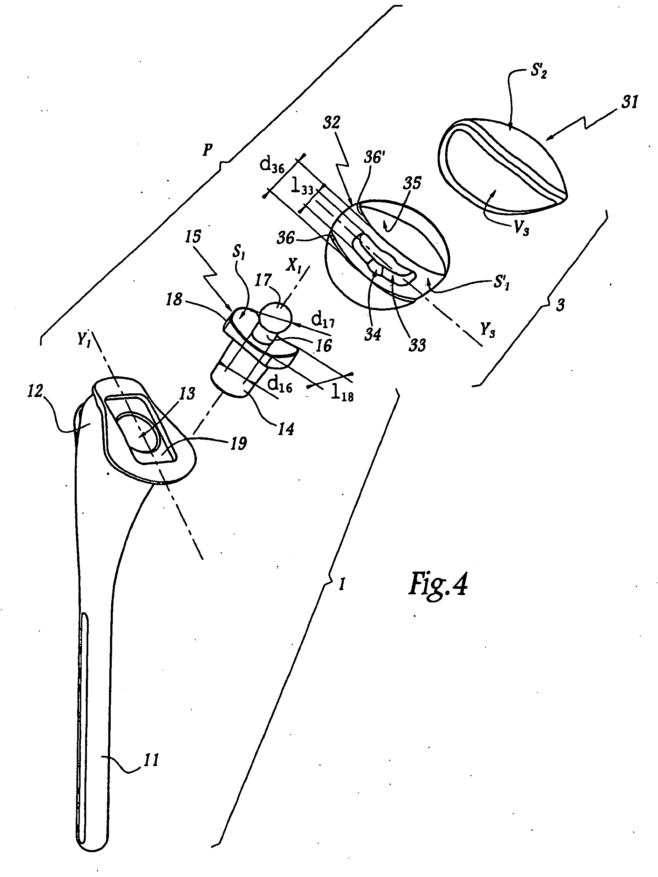

La prothèse P représentée sur les figures 1 à 4 comprend un composant huméral 1 qui inclut une tige 11 destinée à être ancrée dans le canal médullaire de l'humérus H du patient et dont la partie métaphysaire 12 comporte un logement tronconique 13 de réception et de coincement d'une queue 14 d'ancrage d'un organe ou pion 15 destiné à être rapporté et immobilisé sur la tige 11. Cet organe, qui est monobloc, comprend également un col 16 en forme de tige qui relie une partie 17 globalement sphérique à la partie principale de l'organe 15 qui comprend la queue 14. L'organe 15 est également pourvu d'une collerette globalement rectangulaire 18 dont la forme extérieure lui permet d'être engagée dans une dépression 19 globalement rectangulaire ménagée sur la partie 12, autour du logement 13.

On note S₁ la surface concave de la collerette 18 tournée vers la partie 17. On note X₁ l'axe longitudinal des éléments 14 et 16. On note Y₁ un axe longitudinal de la dépression 19 parallèle à ses plus grands côtés et situé à égal distance de ceux-ci. La surface S₁ est un tronçon de cylindre à génératrice droite et à base circulaire, centré sur un axe Z₁ perpendiculaire aux axes X₁ et Y₁, l'axe Z₁ étant disposé globalement selon une direction antéro-postérieure de l'épaule en configuration montée et implantée de la prothèse.

La prothèse P comprend également un composant glénoïdien 2 ancré dans la glène G et définissant une surface concave S₂ dont la concavité est tournée vers l'extérieur de la glène.

Entre les composants 1 et 2 est disposé un composant intermédiaire 3 comprenant une coupelle creuse ou cupule 31 à l'intérieur de laquelle est immobilisée par tout moyen approprié une bague 32 qui définit une ouverture allongée 33 dont la plus grande dimension s'étend selon un axe Y₃ globalement parallèle à l'axe Y₁ en configuration montée de la prothèse.

En partie médiane, l'ouverture 33 est pourvue d'une zone élargie 34 à travers laquelle la partie 17 est destinée à être introduite en force dans un volume V₃ défini à l'intérieur du composant 3, entre la coupelle 31 et la bague 32.

La bague 32 est réalisée en matière plastique, notamment en polyéthylène, ce qui lui permet de se déformer élastiquement au niveau de la zone 34, lors de l'introduction de la partie 17 dans le volume V₃. Pour faciliter cette introduction, la zone 34 est convergente en direction du volume V₃.

Le diamètre d₁₇ de la partie 17, est supérieur à la largeur l₃₃ de l'ouverture 33, y compris au niveau de la zone 34, alors que le diamètre d₁₆ du col 16 est légèrement inférieur à cette largeur. Ainsi, lorsque la partie 17 a été introduite dans le volume V₃, elle résiste à un effort de séparation des éléments 1 et 3 et contribue à la tenue à l'arrachement de la prothèse P. Les valeurs respectives du diamètre d₁₆ et de la largeur l₃₃ sont compatibles avec la translation du col ou tige 16 selon la direction longitudinale Y₃ de l'ouverture 33.

On note S'₂ la surface extérieure convexe de la coupelle 31. On note S'₁ la partie de la surface externe 35 de la bague 32 qui entoure l'ouverture 33. La surface 35 est la surface de la bague 32 tournée à l'opposé du volume V₃.

La surface S'₂ est un tronçon de sphère avec un rayon analogue à celui de la surface S₂, ce qui permet une articulation surfacique des surfaces S₂ et S'₂ l'une sur l'autre.

La surface S'₁ est un tronçon de cylindre à génératrice droite et à base circulaire centré sur un axe Z₃ perpendiculaire à l'axe Y₃ et confondu avec l'axe Z₁ lorsque les surfaces S₁ et S'₁ sont en appui l'une sur l'autre.

Ainsi, l'articulation de l'humérus H par rapport à la glène G a lieu par glissement des surfaces S₁ et S'₁ l'une sur l'autre et des surfaces S₂ et S'₂ l'une sur l'autre.

La surface S'₁ est ménagée en creux par rapport au reste de la surface externe 35 de la bague 32 qui forme une sorte de glissière. La surface S'₁ est définie entre les deux bords 36 et 36' de cette glissière, parallèles à l'axe Y₃ et séparés par une distance d₃₆ légèrement supérieure à la largeur l₁₈ de la collerette 18. Ainsi, la collerette 18 peut être partiellement engagée entre les bords 36 et 36', ce qui contribue au guidage en translation du composant 3 par rapport au composant 1.

La surface interne 39 de la bague 32 a le même rayon de courbure que la surface 35.

Selon une variante non représentée de l'invention, les surfaces S₁ et S'₁ peuvent être des tronçons de sphère au lieu de tronçons de cylindre. Dans ce cas, le guidage en translation de la tige 16 est assuré essentiellement par les bords de la glissière.

Selon une variante non représentée de l'invention, l'axe Y₁-Y'₁ de la dépression 19 peut être disposé selon une direction antéro-postérieure, alors que l'axe Z₁ est disposé selon une direction supéro-inférieure. Une telle variante favorise le mouvement d'abduction.

Selon d'autres variantes non représentées, la partie 17 peut avoir différentes formes, par exemple en tronc de cône.

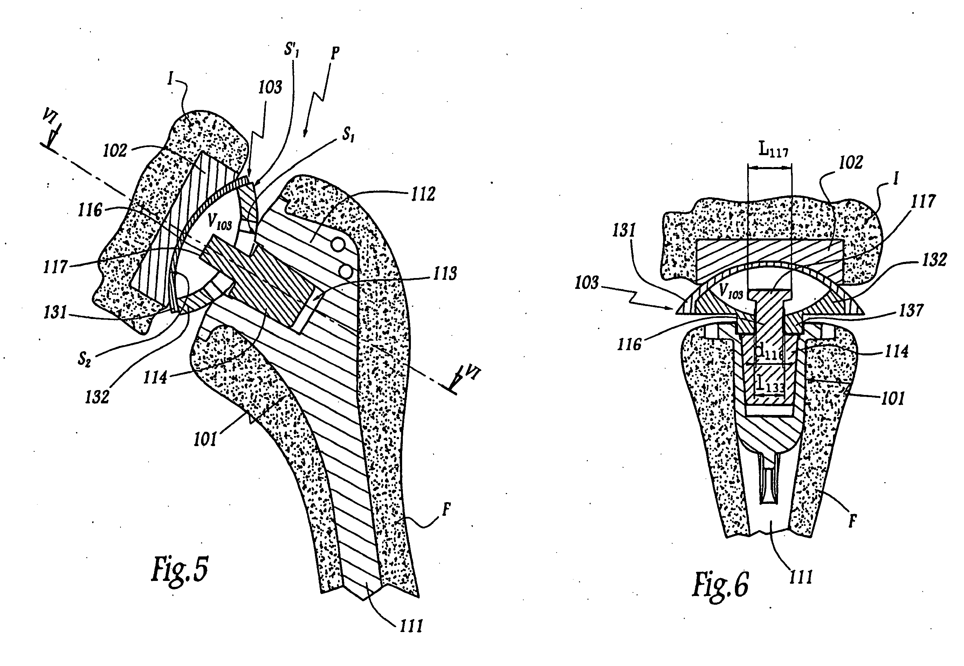

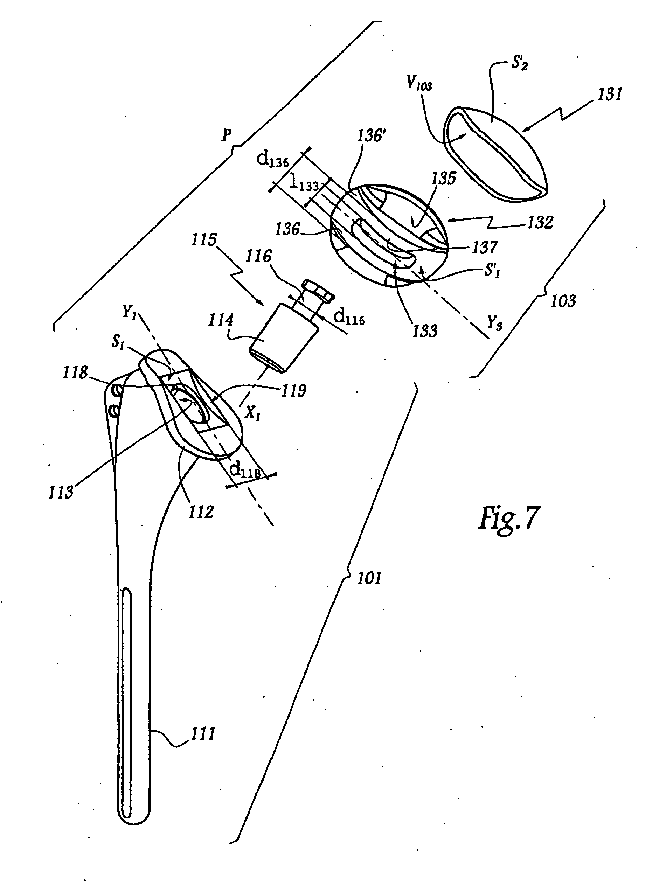

Dans le second mode de réalisation de l'invention représenté aux figures 5 à 7, les éléments analogues à ceux du premier mode de réalisation portent des références identiques augmentées de 100. La prothèse P de ce mode de réalisation est destinée à équiper la hanche d'un patient. Un composant fémoral 101 comprend une tige 111 destinée à être ancrée dans le canal médullaire du fémur F, ainsi qu'une partie métaphysaire 112 dans laquelle est ménagé un logement 113 de réception et de blocage d'une queue 114 d'ancrage d'un organe monobloc 115 qui comprend un col 116 en forme de tige reliant une tête 117 à la queue 114.

Un composant cotyloïdien 102 est fixé dans l'os iliaque I.

Comme précédemment, un composant intermédiaire 103 est formé d'une coupelle ou cupule 131 dans laquelle est clipsée une bague 132 en matière plastique. Une ouverture allongée 133 est ménagée dans la bague 132 et s'étend dans la direction d'un axe Y₃.

En variante, la bague 132 pourrait être métallique.

La largeur l₁₃₃ de l'ouverture 133 est inférieure à la plus grande dimension L₁₁₇ de la tête 117 et supérieure au diamètre d₁₁₆ du col 116 en forme de tige. La plus petite dimension l₁₁₇ de la tête 117 est sensiblement égale au diamètre d₁₁₆, ce qui permet d'introduire la tête 117 dans un volume intérieur V₁₀₃ du composant 103, défini entre la coupelle 131 et la bague 132, en alignant la plus grande dimension de la tête 117 sur l'axe Y₃. Ensuite, moyennant une rotation de l'organe 115 d'environ 90° autour de l'axe X₁ commun aux parties 114 et 116, la tête 117 est retenue dans le volume V₁₀₃.

On note S₂ la surface concave définie par le composant 102. On note S'₂ la surface externe convexe de la coupelle 131. Les surfaces S₂ et S'₂ sont globalement en tronçons de sphère, ce qui permet de créer une articulation entre ces deux surfaces.

Comme dans le premier mode de réalisation, une première surface d'articulation convexe S'₁ est ménagée sur la surface externe 135 de la bague 132, autour de l'ouverture 133.

Une surface convexe S'₁ est formée sur une partie en saillie 137 de la bague 132 dont on note 136 et 136' les bords longitudinaux qui sont parallèles à l'axe Y₃.

Une surface concave S₁ complémentaire de la surface S' est formée par la partie métaphysaire 112 et constitue le fond d'une dépression 119 à section globalement rectangulaire ménagée dans la face exposée 112a de la partie 112, lorsque le composant 101 est en place dans le fémur F.

La dépression 119 est de forme allongée, avec sa plus grande dimension disposée selon un axe Y₁ globalement parallèle à l'axe Y₃ en configuration montée de la prothèse. On note 118 et 118' les bords longitudinaux de la dépression 119. La distance d₁₁₈ entre ces bords est légèrement supérieure à la largeur de la partie 137 qui correspond en fait à la distance d₁₃₆ entre les bords 136 et 136'. Ceci contribue au guidage latéral entre les composants 101 et 103, car la partie 137 est partiellement engagée dans la dépression ou glissière 119 lorsque les composants 1 et 3 sont en appui l'un sur l'autre.

Les surfaces S₁ et S'₁ peuvent être en tronçons de sphère ou en tronçons de cylindre à base circulaire et à génératrice rectiligne.

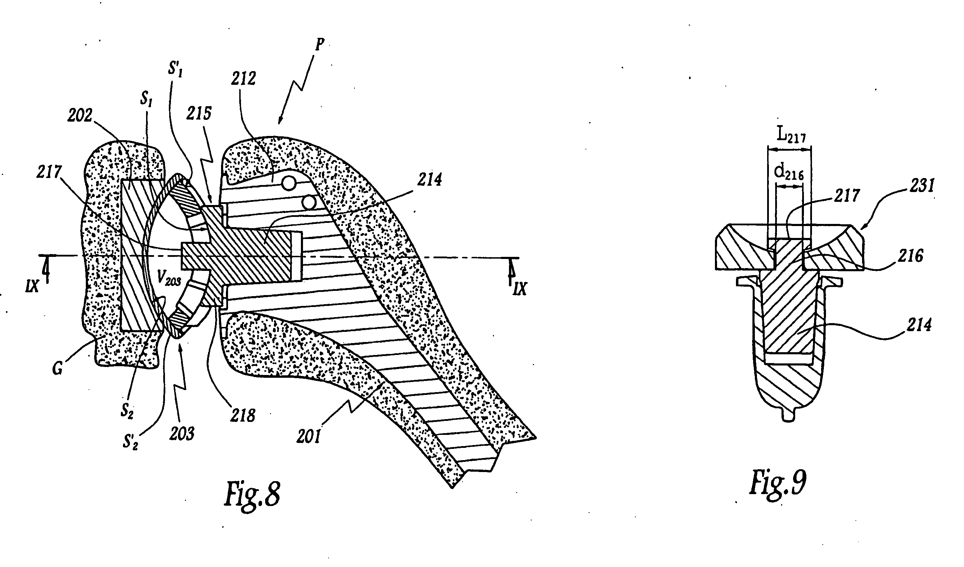

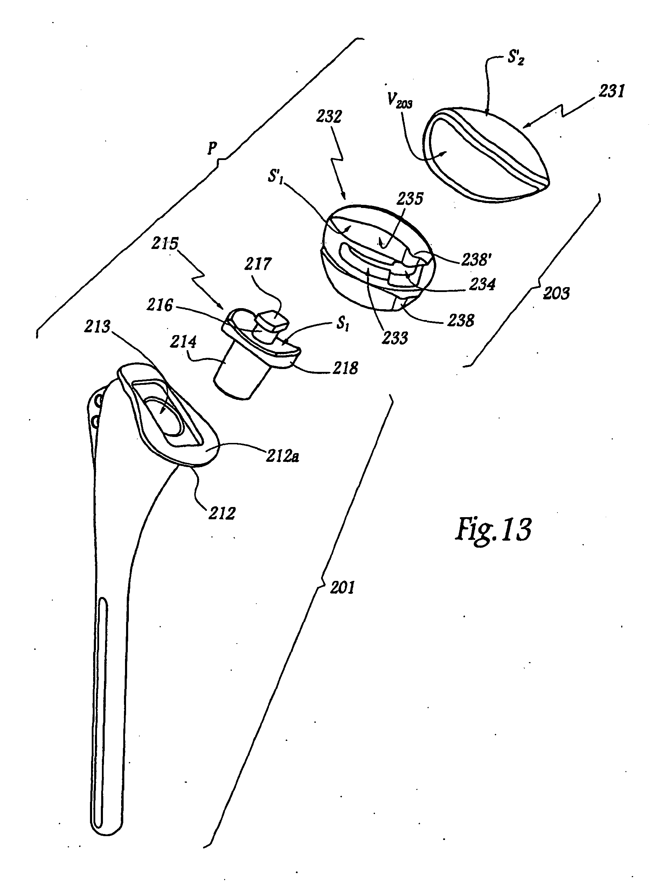

Dans le troisième mode de réalisation de l'invention représenté aux figures 8 à 13, les éléments analogues à ceux du premier mode de réalisation portent des références identiques augmentées de 200. La prothèse P de ce mode de réalisation est destinée à équiper l'épaule d'un patient et comprend un composant huméral 201 présentant sensiblement la même géométrie que le composant huméral du premier mode de réalisation. Un organe 215 est destiné à être ancré par une queue 214 dans un logement 213 ménagé dans la partie métaphysaire 212 du composant 201. L'organe 215 a sensiblement la même géométrie que l'organe 15 du premier mode de réalisation au fait près que sa tête 217 est à section globalement rectangulaire. Comme dans le premier mode de réalisation, l'organe 215 définit une surface d'articulation concave S₁.

Un composant glénoïdien 202 est destiné à être fixé dans la glène et définit une surface S₂ également concave.

Un composant intermédiaire 203, prévu pour être disposé entre les composants 201 et 202, est formé d'une coupelle ou cupule 231 et d'une bague 232 immobilisée sur cette coupelle par tout moyen approprié, par exemple par sertissage. La bague 232 définit une ouverture allongée 233 de réception et de guidage d'un col en forme de tige 216 qui relie la tête 217 à une collerette 218 et à la queue 214, la collerette 218 définissant la surface S₁.

Une surface S'₁ convexe et complémentaire de la surface S₁ est formée sur la surface externe 235 de la bague 232, alors que la surface externe S'₂ de la bague 231 est complémentaire de la surface S₂.

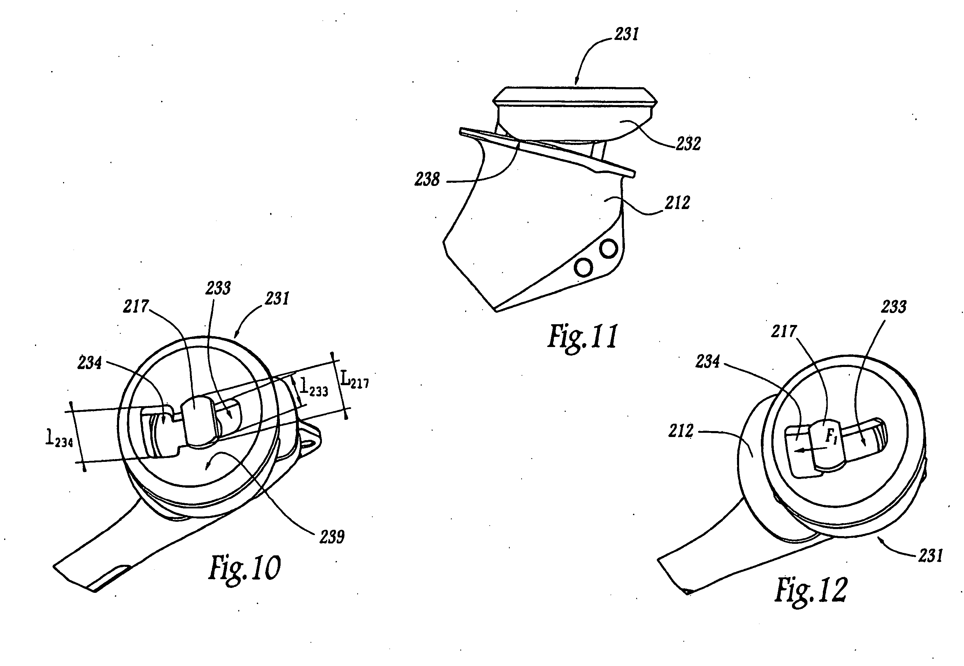

Comme il ressort plus particulièrement des figures 9 à 12 où la coupelle 231 a été omise pour la clarté du dessin, la tête 217 a une longueur L₂₁₇ supérieure à la largeur l₂₃₃ de l'ouverture 233 sur la plus grande partie de sa longueur. Pour permettre l'introduction de la tête 217 dans le volume intérieur V₂₀₃ du composant 203, l'ouverture 233 est pourvue d'une zone élargie 234 ménagée au voisinage d'une de ses extrémités et dont la largeur l₂₃₄ est supérieure à la longueur L₂₁₇. Pour garantir la retenue de la tête 217 dans le volume V₂₀₃, la bague 232 est pourvue de deux talons 238 et 238' destinés à venir en appui contre la face exposée 212a de la partie métaphysaire 212 lorsque le composant 201 est en place dans l'humérus, ce qui bloque la bague 232 dans la configuration des figures 11 et 12 et évite un déplacement de la tête 217 dans le sens de la flèche F₁ à la figure 12, empêchant ainsi un alignement de cette tête avec l'ouverture 204 et, par voie de conséquence, le retrait de la tête 217 du volume V₂₀₃. Ainsi, lorsque la tête 217 a été introduite dans le volume V₂₀₃, elle participe à la tenue de l'assemblage de la prothèse P.

Selon une variante non représentée de l'invention, la fonction des talons 238 et 238' pourrait être remplie par un ou des reliefs ménagés sur la surface interne 239 de la bague 232, par exemple de part et d'autre de l'ouverture 233 et au voisinage de la zone 234. Ce ou ces reliefs empêcherai(en)t alors la tête 217 de se déplacer vers la gauche à partir de sa position de la figure 12.

Selon une autre variante non représentée de l'invention, des reliefs de même fonction que les talons 238 et 238' peuvent être prévus sur la face 212a, voire à la fois sur les composants 201 et 203.

La largeur l₂₃₃ est légèrement supérieure au diamètre d₂₁₆ de la tige 216.

Comme dans le premier mode de réalisation, la surface S'₁ est définie entre des bords parallèles à la direction Y₃ formant une glissière et entre lesquels peut être introduite la collerette 218. De même, les surfaces S₁ et S'₁ peuvent être des tronçons de sphère ou de cylindre, voire d'autres surfaces de forme adaptée.

L'invention a été représentée lors de sa mise en oeuvre avec des prothèses totales d'épaule et de hanche. Elle est cependant applicable avec une prothèse d'épaule dépourvue de composant glénoïdien, la surface d'articulation anatomique de la glène étant utilisée au lieu de la surface S₂ représentée sur les figures. De même, dans le cas d'une prothèse de hanche, la cavité cotyloïdienne naturelle peut être utilisée.

Les caractéristiques des différents modes de réalisation représentés peuvent être combinées entre elles. En particulier, la prothèse du second mode de réalisation peut être adaptée à l'épaule, alors que les prothèses des premier et troisième modes de réalisation peuvent être adaptées à la hanche.

## Revendications

1. Prothèse d'épaule ou de hanche comprenant un premier composante (1;101;201) numéral ou fémoral, présentant une surface d'articulation concave et un deuxième composant (3,103,203), intermédiaire, présentant une première (S'₁) et une seconde (S'₂) surfaces d'articulation convexes destinées à coopérer respectivement avec ladite surface d'articulation concave (S₁) dudit premier composant et avec une surface d'articulation concave glénoïdienne ou cotyloïdienne naturelle ou définie par un troisième composant (2;102;202) glénoïdien ou cotyloïdien, ledit deuxième composant étant creux et définissant un volume de réception (3;103;203) d'une partie (17;117;217) d'un organe (15;115;215) appartenant audit premier composant, ladite partie étant reliée à une partie métaphysaire (12;112;212) dudit premier composant par une tige (16;116;216) traversant une ouverture (33;133;233) menagée dans une bague (32;132;232) dudit second composant, la bague étant montée sur une cupule (31;131;231) dont la surface externe forme la seconde surface d'articulation convexe (S'₂) dudit deuxième composant, **caractérisée en ce que** la première surface d'articulation convexe (S'₁) dudit deuxième composant (3 ; 103 ; 203) est formée sur la surface externe (35, 135, 235) de ladite bague (32 ; 132 ; 232)., autour de ladite ouverture (33 ; 133 ; 233).

2. Prothèse selon la revendication 1, **caractérisée en ce que** ladite ouverture (33 ; 133 ; 233) est allongée, avec une largeur (l₃₃ ; l₁₃₃ ; l₂₃₃) inférieure à la largeur maximale (L₁₇ ; L₁₁₇ ; L₂₁₇) de ladite partie (17 ; 117 ; 217) dudit organe (15 ; 115 ; 215) reçue dans ledit volume (V₃ ; V₁₀₃ ; V₂₀₃), la largeur de ladite ouverture étant légèrement supérieure à la largeur (d₁₆ ; d₁₁₆ ; d₂₁₆) de ladite tige.

3. Prothèse selon l'une des revendications précédentes, **caractérisée en ce que** ladite surface d'articulation concave (S₁) dudit premier composant (1 ; 201) est formée sur une portion (18 ; 218) dudit organe (15 ; 215) monobloc avec ladite partie (17 ; 217) et ladite tige (16 ; 216), ledit organe étant rapporté sur une partie métaphysaire (12 ; 212) dudit premier composant.

4. Prothèse selon la revendication 1 ou 2,
**caractérisée en ce que** ladite surface d'articulation concave (S₁) dudit premier composant (101) est formée sur une partie métaphysaire (112) dudit premier composant.

5. Prothèse selon l'une des revendications précédentes, **caractérisée en ce que** l'une desdites surfaces, parmi ladite première surface convexe (S'₁) et ladite surface concave (S₁) dudit premier composant, est en saillie par rapport au composant (1 ; 103 ; 201) auquel elle appartient, alors que l'autre desdites surfaces, parmi les mêmes deux surfaces, est en retrait par rapport à l'autre composant (3 ; 101 ; 203), la surface en saillie étant bordée par deux bords (18 ; 136, 136' ; 218) globalement parallèles, alors que la surface en retrait est bordée par deux bords globalement parallèles entre lesquels sont disposés les bords de la surface en saillie en configuration montée de la prothèse (P).

6. Prothèse selon l'une des revendications précédentes, **caractérisée en ce que** l'un (3 ; 101 ; 203) desdits premier (1 ; 101 ; 201) et deuxième (3 ; 103 ; 203) composants forme une glissière en creux (36, 36' ; 119) de réception et de guidage d'une partie en saillie (18 ; 137 ; 218) de l'autre compostant (1 ; 103 ; 201), parmi lesdits premier et deuxième composants, ladite première surface d'articulation convexe (S'₁) et ladite surface d'articulation concave (S₁) dudit premier composant étant respectivement formées par le fond de ladite glissière et par la surface d'extrémité de ladite partie en saillie, ou inversement.

7. Prothèse selon l'une des revendications précédentes, **caractérisée en ce que** ladite ouverture (33 ; 233) est pourvue d'une zone élargie (34 ; 234) d'introduction de ladite partie (17 ; 217) dudit organe (15 ; 215) dans ledit volume de réception (V₃ ; V₂₀₃) .

8. Prothèse selon la revendication 7, **caractérisée en ce que** ladite bague (32) est élastiquement déformable au moins au niveau de ladite zone élargie (34), de façon à permettre l'introduction de ladite partie (17) dans ledit volume (V₃).

9. Prothèse selon la revendication 7, **caractérisée en ce que** ladite zone élargie (234) est ménagée à une extrémité de ladite ouverture (233) qui est allongée, l'un au moins (203) desdits premier et deuxième composant étant pourvu d'au moins un relief (238, 238') de blocage, apte à empêcher l'alignement de ladite partie (217) et de ladite zone élargie en configuration montée de la prothèse.

10. Prothèse selon l'une des revendications précédentes, **caractérisée en ce que** ladite première surface d'articulation convexe (S'₁) est globalement en forme de tronc de cylindre centré sur un axe (Y₃) antéro-postérieur de l'articulation en configuration montée et implantée de la prothèse (P).

## Claims

1. Prosthesis for a shoulder or a hip, comprising a first component (1; 101; 201), humeral or femoral, presenting a concave surface of articulation and a second, intermediate, component (3, 103, 203), presenting first (S₁') and second (S₂') convex surfaces of articulation intended to cooperate respectively with the said concave surface of articulation (S₁) of the said first component and with a concave glenoidal or cotyloidal surface of articulation, either natural or defined by a third, glenoidal or cotyloidal, component (2; 102; 202), the said second component being hollow and defining a space (V₃; V₁₀₃; V₂₀₃) for reception of a part (17; 117; 217) of a member (15; 115; 215) belonging to the first component, the said part being joined to a metaphyseal part (12; 112; 212) of the first component by a stem (16; 116; 216) across an opening (33; 133; 233) provided in a ring (32; 132; 232) of the second component, the ring being mounted on a cup (31; 131; 231) of which the exterior constitutes the second convex surface of articulation (S₂') of the said second component, **characterized in that** the first convex surface of articulation (S₁') of said second component (3, 103, 203) is formed on the external surface (35; 135; 235) of the said ring (32; 132; 232), around the said opening (33; 133; 233).

2. Prosthesis according to claim 1, **characterized in that** the said opening (33; 133; 233) is elongate, with a breadth (I₃₃; I₁₃₃; I₂₃₃) less than the maximum breadth (L₁₇; L₁₁₇; L₂₁₇) of the said part (17; 117; 217) of the said member (15; 115; 215) received in the said space (V₃; V₁₀₃; V₂₀₃), the breadth of the said opening being slightly greater than the breadth (d₁₆; d₁₁₆; d₂₁₆) of the said stem.

3. Prosthesis according to any of the foregoing claims, **characterized in that** the said concave surface of articulation (S₁') of the first component (1; 101; 201) is formed on a portion (18; 218) of the said member (15; 215) integral with the said part (17; 217) and the said stem (16; 216), the said member being coupled to a metaphyseal part (12; 212) of the said first component.

4. Prosthesis according to claim 1 or 2, **characterized in that** the said concave surface of articulation (S₁) of the first component is formed on a metaphyseal part (112) of the said first component.

5. Prosthesis according to any of the foregoing claims, **characterized in that** one of the said surfaces out of the said first convex surface (S₁') and the said concave surface (S₁) of the said first component projects with respect to the component (1; 103; 201) to which it pertains, whereas the other of the said surfaces out of the same two surfaces is recessed relative to the other component (3; 101; 203), the projecting surface being bounded by two generally parallel sides (18; 136, 136'; 218), whereas the recessed surface is bounded by two generally parallel sides between which are disposed the sides of the projecting surface in the assembled configuration of the prosthesis (P).

6. Prosthesis according to any of the foregoing claims, **characterized in that** one (3; 101; 203) of the first (1; 101; 201) and second (3; 103; 203) components forms a hollow slideway (36, 36'; 119) for the reception and guidance of a projecting part (18; 137; 218) of the other component (1; 103; 201) of the said first and second components, the said first convex surface of articulation (S₁') and the said concave surface of articulation (S₁) being constituted respectively by the bottom of the said slideway and by the end surface of the said projecting part or *vice versa*.

7. Prosthesis according to any of the foregoing claims, **characterized in that** the said opening (33; 233) is provided with an enlarged region (34; 234) for introduction of the said part (17; 217) of the said member (15; 215) into the said space for reception (V₃; V₂₀₃).

8. Prosthesis according to claim 7, **characterized in that** the said stem (32) is elastically deformable at least at the level of the said enlarged region (34) so as to permit the introduction of the said part (17) into the said space (V₃).

9. Prosthesis according to claim 7, **characterized in that** the said enlarged region (234) is provided at one end of the said opening (233) which is elongate, at least one (203) of the first and second components being provided with at least one raised stop (238, 238') adapted to prevent the alignment of the said part (217) and the enlarged region in the assembled configuration of the prosthesis.

10. Prosthesis according to any of the foregoing claims, **characterized in that** the said first convex surface of articulation (S₁') is generally in the form of a cylindrical trunk centred on a front-to-back axis (Y₃) of articulation in the assembled configuration of the prosthesis (P).

## Patentansprüche

1. Schulter- oder Hüftprothese, die ein erstes Humerus- oder Femur-Bauteil (1; 101; 201), das eine konkave Gelenkfläche hat, und ein zweites Zwischenbauteil (3; 103; 203) aufweist, das eine erste (S'₁) und eine zweite (S'₂) konvexe Gelenkfläche hat, die dazu bestimmt sind, je mit der konkaven Gelenkfläche (S₁) des ersten Bauteils und mit einer natürlichen glenoidalen oder cotyloidalen oder durch ein drittes glenoidales oder cotyloidales Bauteil (2; 102; 202) definierten konkaven Gelenkfläche zusammenzuwirken, wobei das zweite Bauteil hohl ist und ein Aufnahmevolumen (V₃; V₁₀₃; V₂₀₃) für einen Teil (17; 117; 217) eines Organs (15; 115; 215) definiert, das zum ersten Bauteil gehört, wobei der Teil mit einem Metaphysenteil (12; 112; 212) des ersten Bauteils über eine Stange (16; 116; 216) verbunden ist, die eine Öffnung (33; 133; 233) durchquert, die in einem Ring (32; 132; 232) des zweiten Bauteils ausgebildet ist, wobei der Ring auf eine Schale (31; 131; 231) montiert ist, deren Außenfläche die zweite konvexe Gelenkfläche (S'₂) des zweiten Bauteils bildet, **dadurch gekennzeichnet, dass** die erste konvexe Gelenkfläche (S'₁) des zweiten Bauteils (3; 103; 203) auf der Außenfläche (35, 135, 235) des Rings (32; 132; 232) um die Öffnung (33; 133 ; 233) herum geformt ist.

2. Prothese nach Anspruch 1, **dadurch gekennzeichnet, dass** die Öffnung (33; 133; 233) länglich ist, mit einer geringeren Breite (l₃₃; l₁₃₃; l₂₃₃) als die maximale Breite (L₁₇; L₁₁₇; L₂₁₇) des Teils (17, 117; 217) des Organs (15; 115; 215), der im Volumen (V₃; V₁₀₃; V₂₀₃) aufgenommen wird, wobei die Breite der Öffnung geringfügig größer als die Breite (d₁₆; d₁₁₆; d₂₁₆) der Stange ist.

3. Prothese nach einem der vorhergehenden Ansprüche, **dadurch gekennzeichnet, dass** die konkave Gelenkfläche (S₁) des ersten Bauteils (1; 201) auf einem Abschnitt (18; 218) des Organs (15; 215) geformt ist, der einstückig mit dem Teil (17; 217) und der Stange (16; 216) ist, wobei das Organ auf einen Metaphysenteil (12; 212) des ersten Bauteils aufgesetzt ist.

4. Prothese nach Anspruch 1 oder 2, **dadurch gekennzeichnet, dass** die konkave Gelenkfläche (S₁) des ersten Bauteils (101) auf einem Metaphysenteil (112) des ersten Bauteils geformt ist.

5. Prothese nach einem der vorhergehenden Ansprüche, **dadurch gekennzeichnet, dass** eine der Flächen, die erste konvexe Fläche (S'₁) oder die konkave Fläche (S₁) des ersten Bauteils, bezüglich des Bauteils (1; 103; 201) vorsteht, zu dem sie gehört, während die andere Fläche der gleichen zwei Flächen bezüglich des anderen Bauteils (3; 101; 203) zurückgesetzt ist, wobei die vorstehende Fläche von zwei im Wesentlichen parallelen Rändern (18; 136, 136'; 218) umrandet wird, während die zurückgesetzte Fläche von zwei im Wesentlichen parallelen Rändern umrandet wird, zwischen denen die Ränder der vorstehenden Fläche in der montierten Konfiguration der Prothese (P) angeordnet sind.

6. Prothese nach einem der vorhergehenden Ansprüche, **dadurch gekennzeichnet, dass** eines (3; 101; 203) der ersten (1; 101; 201) und zweiten (3; 103; 203) Bauteile eine Hohlschiene (36, 36'; 119) zur Aufnahme und zur Führung eines vorstehenden Teils (18; 137; 218) des anderen Bauteils (1; 103; 201) der ersten und zweiten Bauteilen formt, wobei die erste konvexe Gelenkfläche (S'₁) und die konkave Gelenkfläche (S₁) des ersten Bauteils je vom Boden der Schiene bzw. von der Endfläche des vorstehenden Teils oder umgekehrt geformt werden.

7. Prothese nach einem der vorhergehenden Ansprüche, **dadurch gekennzeichnet, dass** die Öffnung (33; 233) mit einer verbreiterten Zone (34; 234) zur Einführung des Teils (17; 217) des Organs (15; 215) in das Aufnahmevolumen (V₃; V₂₀₃) versehen ist.

8. Prothese nach Anspruch 7, **dadurch gekennzeichnet, dass** der Ring (32) zumindest in Höhe der verbreiterten Zone (34) elastisch verformbar ist, um die Einführung des Teils (17) in das Volumen (V₃) zu erlauben.

9. Prothese nach Anspruch 7, **dadurch gekennzeichnet, dass** die verbreiterte Zone (234) an einem Ende der Öffnung (233) ausgebildet ist, das länglich ist, wobei mindestens eines (203) der ersten und zweiten Bauteile mit mindestens einem Blockierrelief (238, 238') versehen ist, das geeignet ist, die fluchtende Anordnung des Teils (217) und der verbreiterten Zone in der montierten Konfiguration der Prothese zu verhindern.

10. Prothese nach einem der vorhergehenden Ansprüche, **dadurch gekennzeichnet, dass** die erste konvexe Gelenkfläche (S'₁) im Wesentlichen die Form eines Zylinderstumpfs hat, der auf eine von vorne nach hinten verlaufende Achse (Y₃) des Gelenks in der montierten und implantierten Konfiguration der Prothese zentriert ist.
